Europäisches Patentamt
European Patent Office
Office européen des brevets

(19)

(11) Publication number: 0 297 855 A1

(12) # EUROPEAN PATENT APPLICATION

(21) Application number: 88305919.8

(22) Date of filing: 29.06.88

(51) Int. Cl.[4]: C 07 D 295/16
A 61 K 31/445

(30) Priority: 30.06.87 GB 8715335

(43) Date of publication of application: 04.01.89 Bulletin 89/01

(84) Designated Contracting States: AT BE CH DE ES FR GB GR IT LI LU NL SE

(71) Applicant: GLAXO GROUP LIMITED
Clarges House 6-12 Clarges Street
London W1Y 8DH (GB)

(72) Inventor: Collington, Eric William
28 Watton Road
Knebworth Hertfordshire (GB)

Mills, Keith
1 Thunder Court Milton Road
Ware Hertfordshire (GB)

Finch, Harry
31 Newlands
Letchworth Hertfordshire (GB)

Woodings, David Francis
17 Old Forge Close
Stanmore Middlesex (GB)

Hayes, Roger
5 Sandringham Road
Potters Bar Hertfordshire (GB)

(74) Representative: Skailes, Humphrey John et al
Frank B. Dehn & Co. Imperial House 15-19 Kingsway
London WC2B 6UZ (GB)

Claims for the following Contracting States: ES + GR.

(54) **Aminocyclopentyl ethers and their preparation and pharmaceutical formulation.**

(57)

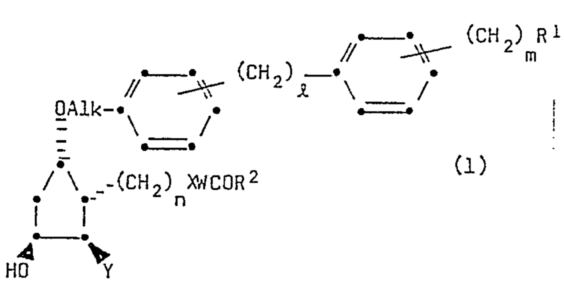

(1)

in which:
W is $C_{1-7}$ alkylene;
X is cis or trans -CH=CH- or $-CH_2CH_2-$;
n is 1 or 2;
Y is a saturated heterocyclic amino group;
Alk is $C_{1-5}$ alkylene;
$\ell$ is zero or 1;
m is zero, 1, 2, 3 or 4;
$R^1$ is a hydroxyl group or a group selected from $-OCOR^3$, $-CO_2R^3$, $-CONR^3R^4$, $-SO_2NR^3R^4$, $-NHCOR^3$, $-NHSO_2R^5$, $-SO_2R^5$, $-SR^5$, $-NR^3R^4$, $-COR^5$, $-NHCONR^3R^4$ and $-NHCSNH_2$; and
$-COR^2$ is an acid, ester, thioester or amide group;
including their salts, solvates and cyclodextrin complexes.

These compounds inhibit blood platelet aggregation and bronchoconstriction and may be formulated for use as antithrombotic or antiasthmatic agent.

EP 0 297 855 A1

## Description

## AMINOCYCLOPENTYL ETHERS AND THEIR PREPARATION AND PHARMACEUTICAL FORMULATION

The endoperoxide prostaglandins $G_2$ and $H_2$ and thromboxane $A_2$ are naturally occurring reactive metabolites of arachidonic acid in human platelets. They are not only potent aggregatory agents but are also constrictors of vascular and bronchial smooth muscle, and therefore substances which antagonise their effects are of considerable interest in human medicine.

In GB-A-2097397, GB-A-2108116, GB-A-2167402 and EP-A-0127930 we have described inter alia aminocyclopentane derivatives represented hereinafter by formula (A)

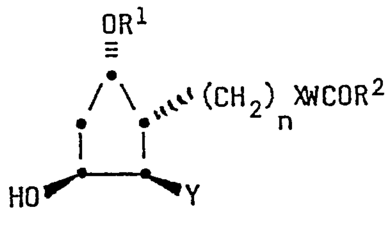

(A)

having endoperoxide and thromboxane antagonist activity. The moiety $R^1$ therein covers a variety of groupings including straight or branched $C_{1-5}$alkyl substituted by phenyl which may itself be substituted by inter alia phenyl (optionally substituted by $C_{1-4}$alkyl, $C_{1-4}$alkoxy or phenyl) or phenyl $C_{1-3}$alkyl.

We have now surprisingly found that substitution of the second phenyl group within $R^1$ above by selected groupings provides novel compounds which have excellent endoperoxide and thromboxane antagonist activity.

The present invention thus provides compounds of the general formula (1)

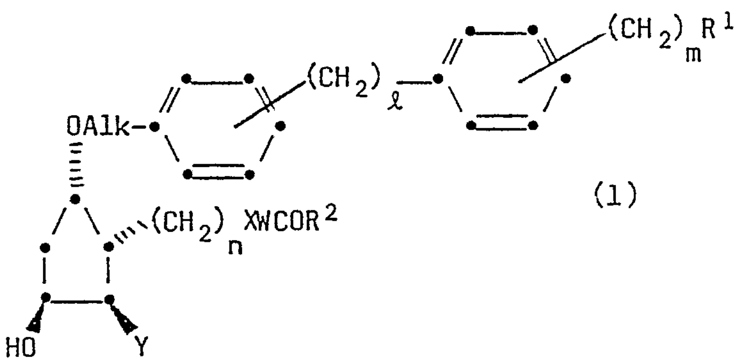

(1)

wherein:

W is straight or branched $C_{1-7}$ alkylene;

X is cis or trans $-CH=CH-$ or $-CH_2CH_2-$;

n is 1 or 2;

Y is a saturated heterocyclic amino group (attached to the cyclopentane ring via the nitrogen atom) which has 5-8 ring members and (a) optionally contains in the ring -O-, -S-, $-SO_2-$, or $-NR^{3a}-$;
and/or (b) is optionally substituted by one or more $C_{1-4}$ alkyl groups;

Alk is a straight or branched $C_{1-5}$ alkylene chain;

$\ell$ is zero or 1;

m is zero, 1, 2, 3 or 4;

$R^1$ is a hydroxyl group or a group selected from $-OCOR^3$, $-CO_2R^3$, $-CONR^3R^4$, $-SO_2NR^3R^4$, $-NHCOR^3$, $-NHSO_2R^5$, $-SO_2R^5$, $-SR^5$, $-NR^3R^4$, $-COR^5$, $-NHCONR^3R^4$ and $-NHCSNH_2$ where $R^3$, $R^{3a}$ and $R^4$, which may be the same or different, represent a hydrogen atom or a $C_{1-4}$ alkyl or $C_{7-10}$ aralkyl group and $R^5$ is a $C_{1-4}$ alkyl group;

$R^2$ is

(a) $-NHR^6$ where $R^6$ is a hydrogen atom or a methyl group or a group $-CH_2CO_2H$ or $-CH_2CONH_2$;

(b) $-AR^7$ where A is -O- or -S- and $R^7$ is phenyl [optionally substituted by $C_{1-4}$alkyl, $C_{1-4}$alkoxy, $C_{1-4}$alkanoyl, methylthio, methylsulphinyl, methylsulphonyl, halogen, $-CO_2R^8$ (where $R^8$ is a hydrogen atom, $C_{1-4}$alkyl or phenyl), $-NHCOR^8$, $-CONR^9R^{10}$ (where $R^9$ and $R^{10}$ may be the same or different and are each a hydrogen atom or $C_{1-4}$alkyl), $C_{1-4}$alkylsulphonylamino, formyl, nitro, cyano, phenyl or $-NR^9R^{10}$];

(c) $-OCH_2COR^{11}$ where $R^{11}$ is phenyl (optionally substituted by a halogen atom, $C_{1-4}$alkyl or $C_{1-4}$alkoxy) or a group $-NR^{12}R^{13}$ (where $R^{12}$ is a hydrogen atom or $C_{1-4}$alkyl and $R^{13}$ is a hydrogen atom, $C_{1-4}$alkyl or phenyl or $NR^{12}R^{13}$ forms a saturated heterocyclic amino group which has 5-7 ring members and optionally contains in the ring -O-);

(d) $-A(CH_2)_pBR^{14}$ where A is as defined above, p is 1-3, B is -O- or -S- and $R^{14}$ is a hydrogen atom, $C_{1-4}$alkyl or a group $-(CH_2)_r$phenyl (where r is 0-3) provided that when p is 1, $R^{14}$ is not a hydrogen atom;

(e) $-A(CH_2)_sR^{15}$ where A is as defined above, s is 2 or 3 and $R^{15}$ is an N-attached $C_{1-4}$dialkylamino, morpholino, piperidino, pyrrolidino, acetylamino or benzoylamino group;

(f) $-OCH(R^{16})_2$ where $R^{16}$ is $-CH_2OH$, $-CHN(CH_3)_2$ or $-CO_2R^{12}$;

(g) $-ACHR^{17}CO_2R^{18}$ where A is as defined above, $R^{17}$ is a hydrogen atom, methyl or phenyl and $R^{18}$ is a hydrogen atom, $C_{1-7}$alkyl, $C_{5-7}$cycloalkyl or phenyl;

(h) $-OCH_2OCOR^{19}$ where $R^{19}$ is $C_{1-4}$alkyl, methoxy or phenyl;

(i) $-OCH_2SCOR^{20}$ where $R^{20}$ is $C_{1-4}$alkyl;

(j) $-AR^{21}$ where A is as defined above and $R^{21}$ is pyridinyl;

(k) $-OR^{22}$ where $R^{22}$ is a hydrogen atom, $C_{1-6}$alkyl, $C_{7-10}$aralkyl, $C_{3-6}$alkenyl, $C_{3-6}$alkynyl, $C_{5-7}$cycloalkyl (optionally substituted by one or more $C_{1-4}$alkyl groups), $-CH_2CCl_3$, furanylmethyl or thienylmethyl;

(l) $-OCH_2CR^{23}R^{24}CH_2OH$ where $R^{23}$ is -OH or $-CH_2OH$ and $R^{24}$ is a hydrogen atom, $C_{1-4}$alkyl or $-CH_2OH$;

(m)

(m) 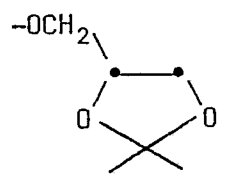 ; or

(n) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; and the physiologically acceptable salts, solvates and cyclodextrin complexes thereof.

The structural formulae herein are to be understood to include the enantiomers of each of the compounds concerned as well as mixtures of the enantiomers including racemates, even though the precise structure as set out only relates to one enantiomer.

Suitable physiologically acceptable salts of the compounds of general formula (1) include acid addition salts derived from inorganic and organic acids, such as hydrochlorides, hydrobromides, sulphates, phosphates, maleates, tartrates, citrates, benzoates, 2-chlorobenzoates, p-toluenesulphonates, methanesulphonates, salicylates, fumarates, lactates, hydroxynaphthalenecarboxylates (eg. 1-hydroxy or 3-hydroxy-2-naphthalenecarboxylates) or furoates. When $R^1$ is a hydrogen atom, the compounds may also form salts with suitable bases. Examples of such salts are alkali metal (eg. sodium and potassium), alkaline earth metal (eg. calcium or magnesium), ammonium and substituted ammonium (eg. dimethylammonium, triethylammonium, 2-hydroxyethyldimethylammonium, piperazinium, N,N-dimethylpiperazinium, piperidinium, ethylenediaminium and choline).

The heterocyclic amino group Y may for example have a 5, 6 or 7-membered ring, e.g. pyrrolidino, piperidino, morpholino, piperazino, thiomorpholino, 1,1-dioxothiomorpholino, homomorpholino and hexamethyleneimino. The carbon atoms of the heterocyclic rings may for example be substituted by methyl, ethyl or butyl. Examples of the optional substituent $R^{3a}$ which may be present on the second nitrogen atom in the ring are methyl, ethyl, butyl, benzyl and phenethyl.

In general Y is preferably a saturated heterocyclic amino group which has 5, 6 or 7 ring members (i.e. pyrrolidino, piperidino or hexamethyleneimino), optionally substituted by one or two $C_{1-4}$ alkyl (particularly methyl) groups, e.g. 4-methylpiperidino. Especially preferred Y groups are piperidino and hexamethyleneimino.

Alk may be, for example, a straight or branched $C_{1-3}$ alkyl chain (eg methylene, ethylene or propylene). Alk preferably represents methylene or propylene.

In the group $-(CH_2)_nXWCOR^2$, n is preferably 2 and X is preferably cis -CH=CH-.

W may contain for example 1-5 carbon atoms in a straight or branched chain, and may be for example $-(CH_2)_4-$ or particularly $-(CH_2)_2-$ when n is 2, or $-(CH_2)_3-$ when n is 1.

In general, the compounds of formula (1) in which the carbon atom carrying the $-(CH_2)_nXWCOR^2$ group is in the R configuration (and mixtures containing this isomer) are preferred.

The group

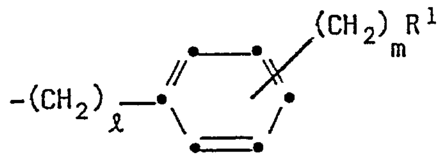

may be attached at the ortho, meta or para position of the phenyl group in the rest of the molecule. Preferably $\ell$ represents zero and the group

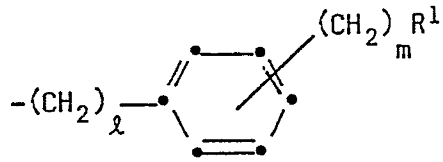

is preferably attached at the meta or, more preferably, the para position. The moiety m is preferably zero, 1 or 2.

Examples of the group $R^1$ include hydroxy, $-OCOR^3$, $CO_2R^3$, $-CONR^3R^4$, $-NHCOR^3$, $-NHSO_2R^5$, $-SO_2R^5$, $-SO_2NR^3R^4$, $-NR^3R^4$ and $-NHCONR^3R^4$.

When $R^3$ or $R^4$ is a $C_{1-4}$ alkyl group the $C_{1-4}$ alkyl group may be straight or branched and may be, for example, a methyl, ethyl, n-propyl, i-propyl, n-butyl- or t-butyl. When $R^3$ or $R^4$ is a $C_{7-10}$ aralkyl group it may be, for example, benzyl or phenethyl. $R^3$ and $R^4$ preferably independently represent hydrogen atoms or methyl groups. Examples of the group $R^5$ include methyl, ethyl, n-propyl, i-propyl, n-butyl- and t-butyl.

In the $R^2$ groups, A (where present) is preferably -O-. In $R^2$ groups of the type (a) $R^6$ is preferably hydrogen, $-CH_2CO_2H$ or $-CH_2CONH_2$, especially hydrogen. In $R^2$ groups of the type (b) $R^7$ is preferably phenyl [optionally substituted by $C_{1-4}$alkyl, $C_{1-4}$alkoxy, $C_{1-4}$alkanoyl, methylsulphonyl, $CO_2R^8$ (where $R^8$ is a hydrogen atom or $C_{1-4}$alkyl), $-NHCOR^8$ (where $R^8$ is $C_{1-4}$alkyl) or $-CONR^9R^{10}$]. In $R^2$ groups of the type (c) $R^{11}$ is preferably phenyl, halophenyl, $-NH_2$, $-N(CH_3)_2$, $-NHR^{13}$ (where $R^{13}$ is methyl, ethyl or phenyl) or $-NR^{12}R^{13}$ is morpholino. In $R^2$ groups of the type (d) p is preferably 1 or 2 and $R^{14}$ is preferably $C_{1-4}$alkyl (e.g. methyl) or phenylmethyl. In $R^2$ groups of the type (e) $R^{15}$ is preferably $C_{1-4}$dialkylamino, morpholino or acetylamino. In $R^2$ groups of the type (f) $R^{12}$ may be, for example, ethyl. In $R^2$ groups type (g) $R^{17}$ is preferably methyl and $R^{18}$ is preferably $C_{1-7}$alkyl or $C_{5-7}$cycloalkyl. In $R^2$ groups of the type (h) $R^{19}$ is preferably $C_{1-4}$alkyl (e.g. methyl). In $R^2$ groups of the type (i) $R^{20}$ is preferably methyl. In $R^2$ groups of the type (k) $R^{22}$ is preferably hydrogen, $C_{1-3}$alkyl (e.g. methyl), benzyl, phenethyl, $C_{3-6}$alkenyl (e.g. allyl), $C_{3-6}$alkynyl (e.g. propynyl) or thienylmethyl, especially hydrogen. In $R^2$ groups of the type ($\ell$) $-CR^{23}R^{24}-$ is preferably -CHOH- or $-C(CH_3)(CH_2OH)-$.

Preferred $R^2$ groups are hydroxy, methoxy, $-OCH_2OCOCH_3$, $-OCH_2SCH_3$, $-O(CH_2)_3NHCOCH_3$, $-OCH_2CONH_2$, 4-acetamidophenoxy, allyloxy, $-OCH_2CON(CH_3)_2$ and $-OCH_2CH(OH)CH_2OH$. Compounds in which $R^2$ represents hydroxy are particularly preferred.

A particular group of compounds of formula (1) are those of formula (1a)

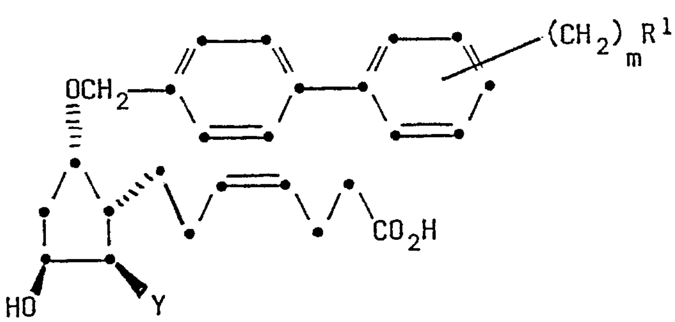

(1a)

and the physiologically acceptable salts, solvates and cyclodextrin complexes thereof, wherein m is zero, 1 and or 2, Y is saturated heterocyclic amino group which has 5, 6 or 7 ring members (e.g. piperidino) and $R^1$ is as previously defined. Preferred compounds of formula (1a) are those in which $R^1$ represents -OH, $-CO_2H$, $-CONH_2$, $-NHCOCH_3$-, $-NHSO_2CH_3$, $-SO_2NHCH_3$, $-NHCONH_2$ or $-SO_2CH_3$, especially -OH. Compounds of formula (1a) in which the group $-(CH_2)_mR^1$ is attached at the ortho position of the phenyl group in the rest of the molecule are particularly preferred.

Compounds of formula (1) have excellent endoperoxide and thromboxane antagonist activity and

consequently inhibit blood platelet aggregation, bronchoconstriction and vasoconstriction. A test to determine inhibition of blood platelet aggregation is as described by Lumley and Humphrey (J. Pharmacol. Methods, 1981, 6, 153-166) using collagen as the pro-aggregatory agent.

The ability of the compounds of the invention to inhibit vasoconstriction or bronchoconstriction is determined using the relevant isolated tissue (eg. spirally cut rat aortic strip or guinea-pig lung parenchymal strip) by measuring the effect of the compound to be tested on the contraction of the tissue to [1R-[1α,4α,5β(Z), 6α(1E,3S*)]]-7-[6-(3-hydroxy-1-octenyl)-2-oxabicyclo-[2.2.1]hept-5-yl]-5-heptenoic acid (U-46619).

The present compounds are thus of interest for use in human and animal medicine, more particularly for use in the treatment or prophylaxis of conditions mediated by thromboxane $A_2$. The compounds of formula (1) may find particular use in the treatment of asthma, and in the treatment and prophylaxis of occlusive vascular disease, including myocardial infarction, cardiac fatalities, unstable angina, transient ischaemic attacks and cerebral infarction, atherosclerosis and vessel wall disease, peripheral vascular disease, nephropathy, retinopathy, postoperative thrombosis and pulmonary embolism. The compounds are also of interest for use in renal dialysis and cyclosporin A-induced nephrotoxicity. In addition, the compounds of the invention are useful in the prophylaxis of peri- and postoperative complications following organ transplantation (particularly cardiac and renal), coronary artery bypass, peripheral artery bypass, angioplasty, thrombolysis and endarterectomy.

The compounds are also of potential use in the treatment of adult respiratory distress syndrome and the prevention of relapse of healed peptic ulcers.

The compounds may be formulated in a conventional manner for use with one or more pharmaceutical carriers.

For oral administration, the pharmaceutical composition may take the form of, for example, tablets, capsules, powders, solutions, syrups, or suspensions prepared by conventional means with acceptable excipients.

The compounds may be formulated for parenteral administration by injection or continuous infusion. Formulations for injections may be presented in unit dosage form in ampoules, or in multi-dose containers, with an added preservative. The compositions may take such forms as suspensions, solutions or emulsions in oil or aqueous vehicles, and may contain formulatory agents such as suspending, stabilising and/or dispersing agents. Alternatively, the active ingredient may be in powder form for reconstitution before use with a suitable vehicle eg. sterile pyrogen-free water.

For administration by inhalation the compounds are conveniently delivered in the form of an aerosol spray presentation from pressurised packs or a nebuliser, or as cartridge from which the powdered composition may be inhaled with the aid of a suitable device. In the case of a pressurised aerosol the dosage unit may be determined by providing a valve to deliver a metered amount.

The precise dose administered will of course depend on the age and condition of the patient, the specific condition to be treated and the mode of administration.

For use as antithrombotic agents, the compounds are preferably administered orally, for example in amounts of 0.05 to 10mg/kg body weight, 1 to 4 times daily.

For use in the treatment of asthma, the compounds may also be administered orally in amounts of 0.05 to 10mg/kg body weight, 1 to 4 times daily. Preferably however they are administered by inhalation at doses varying from 0.02 to 30mg, preferably 0.02 to 3mg, 1 to 4 times daily.

The compounds of the invention may, if desired, be administered in combination with one or more other therapeutic agents such as a thromboxane synthase inhibitor or an antiasthmatic agent.

Suitable methods for preparing compounds of formula (1) are described below, the groups $R^1$, $R^2$, W, X, Alk, Y, $\ell$, m and n being as defined above except where otherwise indicated. It will be appreciated that the following reactions may require the use of, or conveniently may be applied to, starting materials having protected functional groups, and deprotection might thus be required as a final step to yield a compound of the invention. Protection and deprotection of functional groups may be effected using conventional techniques. Thus, for example, amino groups may be protected by acylation, subsequent deacylation being effected when desired by hydrolysis using for example an acid such as hydrochloric acid. Hydroxyl groups may be protected using conventional hydroxyl protecting groups, for example as described in 'Protective Groups in Organic Chemistry', Ed. J. F. W. McOmie (Plemun Press, 1973) or 'Protective Groups in Organic Synthesis', by Theodora W. Greene (John Wiley and Sons, 1981). Examples of suitable protecting groups include heterocyclic groups such as tetrahydropyranyl which may be removed by hydrolysis under acidic conditions.

According to a process (a) compounds of formula (1) may be prepared by reacting corresponding compounds of formula (2)

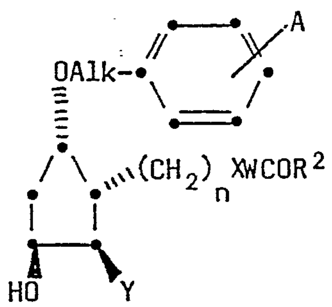

(2)

(where A is a displaceable atom or group) or salts thereof to replace the moiety A with the group

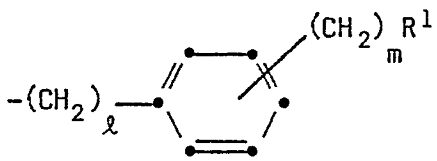

Displaceable atoms or groups represented by the moiety A include any conventional leaving group such as halogen (e.g. chlorine, bromine or iodine), triflate or a phosphate ester (e.g. diethylphosphate).

Replacement of the moiety A by a group

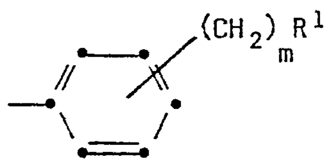

to provide a compound of formula (1) in which $\ell$ is zero may be effected by a coupling reaction of a compound of formula (2) or a salt thereof with a compound of formula (3)

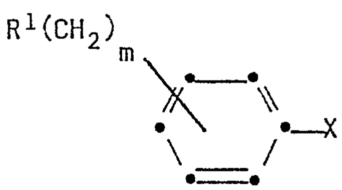

(3)

[where X is an atom or group as defined above for A or X is a group $-B(OH)_2$ or X is a suitable metal atom or metal-containing group such as Li, Cu, MgHal, ZnHal or $SnR'_3$ or X is a group $SiR'_3$ (wherein Hal is a halogen atom, e.g. chlorine, bromine or iodine, and R′ is a $C_{1-6}$alkyl group, e.g. methyl or n-butyl, or an aryl group, e.g. phenyl)].

In a particular embodiment of process (a) compounds of formulae (2) and (3) are reacted wherein A represents a halogen atom (e.g. bromine) and X represents a group $-B(OH)_2$. The coupling reaction may conveniently be carried out in the presence of a suitable transition metal catalyst such as a palladium (O) or palladium (II) catalyst, for example $PdL_4$ or $PdCl_2L_2$ (where L is a phosphine ligand such as triphenylphosphine or tritolylphosphine) in a suitable solvent such as an ether (e.g. 1,2-dimethoxyethane or tetrahydrofuran), a hydrocarbon, for example an aromatic hydrocarbon (e.g. benzene), or a dipolar aprotic solvent such as N,N-dimethylformamide containing an appropriate base which may be, for example, a carbonate, bicarbonate or hydroxide of an alkali or alkaline earth metal (e.g. aqueous sodium carbonate) or a suitable amine such as a tertiary amine (e.g. triethylamine). The reaction may be effected at any suitable temperature up to and including reflux, for example in the range 20°-120°C and preferably in the range 60°-80°C. Ultrasonic techniques or microwave may also be used to facilitate the reaction. The coupling reaction is preferably carried out in the

presence of the catalyst $(Ph_3P)_4Pd$.

When A and X in formulae (2) and (3) are conventional leaving groups as defined for A in formula (2) the coupling reaction may be carried out in a single step in the presence of a suitable transition metal catalyst (e.g. a palladium or nickel catalyst) and under reducing conditions (e.g. using a reducing agent such as zinc metal or hydrazine or by electrolytic reduction). Suitable palladium catalysts include palladium-on-charcoal, a palladium (II) chloride-mercury (II) chloride couple, $PdL_4$ and $PdCl_2L_2$ (where L is as defined above). Suitable nickel catalysts include nickel (II) chloride and $NiCl_2L_2$ (where L is as defined above). The specific conditions for effecting the desired conversion will, of course, depend on the particular values of A and X in formulae (2) and (3) respectively. However, the conditions referred to in the following publications may be suitable for present purposes : Chem. Letters 1986, p. 407; J. Org. Chem. 1986, p. 2627; Tetrahedron Letters 1977, p. 4089; Synthesis 1978, p. 537; Bull. Chem. Soc. Japan 1980, 53, p. 1767 and Tetrahedron Letters 1985, p. 1655.

When X in formula (3) is a conventional leaving group as defined for A in formula (2), the compound of formula (2) may first be converted to a compound of formula (2a)

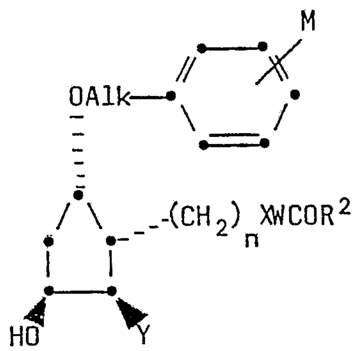

(2a)

[where M is a suitable atom or metal-containing group such as Li, Cu, MgHal, ZnHal, HgHal or $SnR'_3$ or X is a group $SiR'_3$ (wherein Hal and R′ are as previously defined)] and then the compound of formula (2a) reacted with an appropriate compound of formula (3) to give the desired product.

Compounds of formula (2a) may be prepared by treating a compound of formula (2) in which A represents a conventional leaving group such as halogen (e.g. chlorine or, more especially, bromine or iodine), triflate or a phosphate ester (e.g. diethylphosphate) with a reagent capable of introducing the moiety M. Suitable reagents and conditions for effecting the desired conversion are known in the art (cf. J. Am. Chem. Soc. 1987, p. 8056; J. Org. Chem. 1984, p. 5280; Chem. Letters 1981, p. 829; J. Organometal. Chem. 1983, p. 551; Tetrahedron Letters 1987, p. 4715 and Tetrahedron Letter 1983, p. 4895). Thus, for example, a compound of formula (2a) in which M is ZnHal may be prepared from a corresponding halo compound of formula (2) in which A is a halogen atom (e.g. bromine or iodine) by reaction with activated zinc. A compound of formula (2a) in which M is Cu may be prepared from a corresponding halo compound of formula (2) in which A is a halogen atom (e.g. bromine or iodine) by reaction with Riecke copper. A compound of formula (2a) in which M is MgHal may be prepared from a corresponding halo compound of formula (2) in which A is a halogen atom (e.g. bromine or iodine) by reaction with magnesium or with $MgHal_2$ (where Hal is as defined above) in the presence of lithium. A compound of formula (2a) in which M is Li may be prepared from a corresponding halo compound of formula (2) in which A is a halogen atom (e.g. bromine or iodine) by reaction with a suitable organolithium reagent (e.g. n-butyllithium). A compound of formula (2a) in which M is $SnR'_3$ or $SiR'_3$ may be prepared from a corresponding compound of formula (2) in which A is a conventional leaving group such as a halogen atom (e.g. bromine or iodine), triflate or a phosphate ester (e.g. diethylphosphate) by reaction with either $R'_3Sn-SnR'_3$ or $R'_3Si-SiR'_3$ in the presence of a suitable palladium catalyst. Alternatively, $Al(SiR'_3)_3$ and a catalyst $NiCl_2L_2$ (where R′ and L are as defined above) may be used to prepare a compound of formula (2a) in which M is $SiR'_3$.

The resulting compound of formula (2a) is then treated with a compound of formula (3) in which X is a conventional leaving group such as halogen (e.g. bromine or iodine), triflate or a phosphate ester (e.g. diethylphosphate). The coupling reaction may conveniently be effected in the presence of a suitable transition metal catalyst such as a palladium or nickel catalyst (e.g. $PdL_4$, $PdCl_2L_2$, $NiCl_2$ or $NiCl_2L_2$, where L is as defined previously) in a suitable solvent such as an ether (e.g. diethyl ether, tetrahydrofuran or 1,2-dimethoxyethane), hexamethylphosphoramide, dimethylformamide, dioxane, acetonitrile or an aromatic hydrocarbon (e.g. benzene). The specific conditions for effecting the desired reaction will, of course, depend on the particular values of M and X in formulae (2a) and (3) respectively. However, the conditions referred to in the following publications may be suitable for present purposes : Comprehensive Organometal. Chem. volume 8, p. 910; Current Trends in Organic Synthesis (Pergamon Press 1982) p. 269; J. Am. Chem. Soc. 1941, 63, p. 2316; J. Organometal. Chem. 1984, 267, Cl; J. Am. Chem. Soc. 1987, 109, p. 5479; J. Org. Chem. 1983, p. 1333; Tetrahedron Letters 1986, p. 4407; J. Am. Chem. Soc. 1979, 101, p. 4992 and J. Organometal. Chem. 1983, 250,

p. 551.

When X in formula (3) is a metal or metal-containing group as defined previously, the coupling reaction may be carried out with a compound of formula (2) in which A is a conventional leaving group as defined above in a single step using the conditions described above for the coupling of compounds of formulae (2a) and (3).

It is to be understood that the use of stoichiometric amounts of the transition metal "catalyst" may be advantageous in some of the aforementioned coupling reactions.

Intermediates of formula (3) are either known compounds or can be prepared from known compounds using methods analagous to those used to prepare the known compounds of formula (3).

The intermediates of formula (3) in which X is a group $-B(OH)_2$ are either known compounds or may be prepared by methods described by W.J. Thompson et.al. in J. Org. Chem., 1984, 49, 5237.

The boronic acids of formula (3) may be formed in situ under the conditions of the coupling reaction described hereinbefore using the corresponding anhydrides of formula (4) or (4a) which are known classes of compounds described by H.R. Snyder et.al in J. Amer. Chem. Soc., 1958, 80, 3611 and F. R. Bean et. al. in J. Amer. Chem. Soc., 1932, 54, 4415.

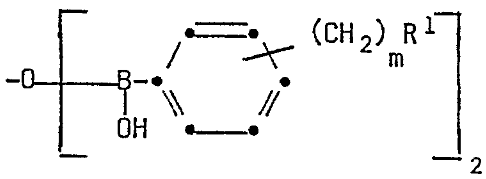

(4)

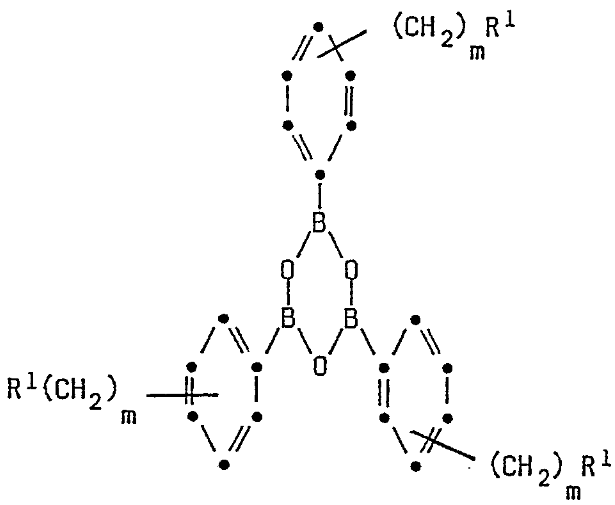

(4a)

The boronic acid of formula (3) where $-(CH_2)_mR^1$ is $2-CH_2OH$ is formed in situ from the known cyclic ester of formula (5)

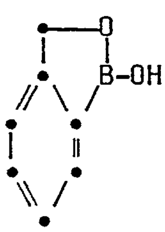

(5)

Where suitable, the group $R^1$ may be protected before the preparation of the corresponding boronic acid (3). Thus, for example, a hydroxyl group may be protected as a silyl ether eg as a t-butyldimethyl silyl ether.

In a further embodiment, compounds of formula (1) in which $\ell$ is 1 may be prepared by reacting a compound of formula (2) (where A is a displaceable atom or group as previously defined) with a suitable organometallic reagent such as a tin containing reagent of formula (6)

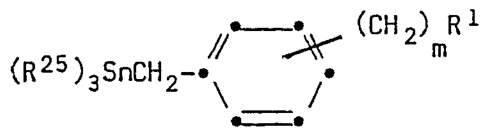

(6)

(where $R^{25}$ is a $C_{1-4}$ alkyl group such as n-butyl) or a zinc containing reagent of formula (7)

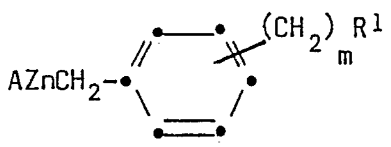

(7)

(where A is a halogen atom, e.g. bromine) in the presence of transition metal catalyst such as $(Ph_3P)_4Pd$, or a pre-reduced nickel acetylacetonate $-Ph_3P$ complex in a suitable solvent such as an ether (e.g. tetrahydrofuran), $[(CH_3)_2N]_3PO$ or a mixture of these, at a suitable temperature, preferably ambient temperature.

The organometallic reagents used in the above processes are either known compounds or may be prepared by analogous methods to those used for the preparation of the known compounds. Where suitable the group $R^1$ may be protected, eg before the preparation of the organometallic reagents (6) and (7).

The skilled chemist will appreciate that process (a) may not be suitable for preparing certain compounds of formula (1), for example those compounds in which $R^2$ contains a halogen atom. Where such compounds are desired one of the alternative process varients described hereinafter may conveniently be used.

In a further process (b) the compounds of formula (1) in which $R^2$ is a hydroxyl group and $\ell$ is zero may be prepared by reacting a boronic acid derivative of formula (8)

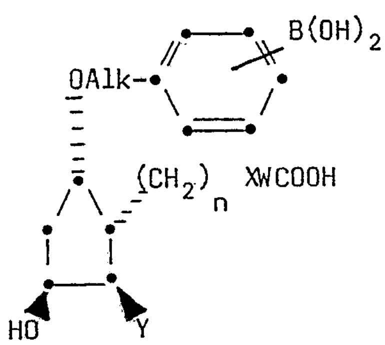

(8)

with a halobenzene of formula (9)

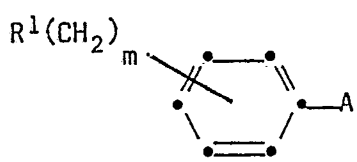

(9)

(where A is a halogen atom, e.g. bromine) under the conditions described in process (a) above for the reaction between a compound of formula (2) in which A is a halogen atom, e.g. bromine, and a compound of formula (3) in which X is a group $-B(OH)_2$.

The intermediates of formula (8) may be prepared by reaction of halobenzenes of formula (2) where $R^2$ is hydroxy with an organolithium reagent such as n-butyl lithium in a solvent such as an ether (e.g. tetrahydrofuran) at low temperature (e.g -100 to -70°C) and a boron reagent such as tri-isopropylborate.

The above conditions may yield the desired boronic acid or the corresponding boronic acid anhydride. If the anhydride is prepared then this compound may be used in the coupling reaction; the corresponding boronic acid may then be formed in situ under the conditions of the coupling reaction.

The intermediates of formula (9) are either known compounds or may be prepared by methods used for the preparation of the known compounds of formula (9).

Processes (c)-(g) below describe methods for the preparation of compounds of formula (1) by interconversion or simple derivatisation.

(c) Compounds of formula (1) containing a group -COOH may be prepared by hydrolysis of a corresponding ester (e.g. a $C_{1-6}$ alkyl ester such as a methyl or t-butyl ester) for example using a base such as sodium hydroxide or potassium hydroxide in a suitable solvent (e.g. an alcohol such as methanol or ethanol) at a suitable temperature up to reflux.

(d) Compounds of formula (1) in which the group $COR^2$ is a carboxylic acid ester or thioester function may be prepared by esterification of the corresponding carboxylic acid or thioacid as appropriate. Conventional esterification techniques may be used, for example by reaction with an appropriate alcohol in the presence of a mineral acid such as hydrochloric acid or sulphuric acid or by reaction with an appropriate halide in the presence of a suitable base, e.g. potassium t-butoxide or potassium carbonate or a sterically hindered amine such as diisopropylethylamine, triethylamine or dicyclohexylamine. The thioacid starting material may be prepared in situ by treating a reactive derivative of the corresponding acid (e.g. a mixed anhydride) with a hydrosulphide (e.g. NaHS).

(e) Compounds of formula (1) in which X is trans -CH=CH- may be prepared by isomerising the corresponding cis compound. The isomerisation may for example by effected by treatment with p-toluene sulphinic acid in dioxan (e.g. at reflux) or azobisisobutyronitrile and thiophenol, using for example a hydrocarbon solvent (e.g. benzene) and any suitable temperature up to reflux.

(f) Compounds of formula (1) in which X is a $-CH_2CH_2-$ group may be prepared by reduction of the corresponding compound in which X is a cis or trans -CH=CH- group. Suitable methods of reduction include hydrogen in the presence of a catalyst such as palladium, on a support (e.g. carbon). Suitable solvents include esters such as ethyl acetate and alcohols such as ethanol and methanol.

(g) Compounds of formula (1) in which the group $COR^2$ is a carboxylic acid amide function may be prepared by amidation of the corresponding carboxylic acid. Conventional methods for converting carboxylic acids to amides may be used; for example by reacting a suitable derivative of the carboxylic acid (e.g. a mixed anhydride) with ammonia or a suitable amine $R^6NH_2$ in a suitable solvent such as acetone or acetonitrile.

(h) Where salts of compounds of formula (1) are desired such salts may be formed using conventional methods, for example by treatment with an acid or with a base. Salt formation may be effected, for example, in a suitable solvent such as an ether (e.g. diethyl ether), a nitrile (e.g. acetonitrile), a ketone (e.g. acetone), a halogenated hydrocarbon (e.g. chloroform or dichloromethane), an ester (e.g. ethyl acetate or isopropyl acetate) or an alcohol (e.g. methanol, ethanol or isopropanol). Salts may also be formed by conversion of one salt of a compound of the invention into another, e.g. by ion exchange using conventional methods.

Cyclodextrin complexes of compounds of formula (1) may be prepared from a compound of formula (1) or a salt (e.g. the hydrochloride salt) thereof by dissolving the compound of formula (1) or a salt thereof in water or an organic solvent which is miscible with water (e.g. an alcohol such as methanol) and adding to the solution a solution of α-, β- or γ-cyclodextrin (or a hydrate thereof) or a mixture of two or three of them together in water and/or an organic solvent which is miscible with water. The reaction may conveniently take place at any temperature in the range from 0° to 80°C. However, the mixture is preferably kept at room temperature and the desired complex obtained by concentrating the mixture under reduced pressure or by allowing the mixture to cool.

The intermediates of formula (2) where $R^2$ is a $C_{1-6}$ alkoxy or $C_{7-10}$ aralkoxy group, n is 2 and X is cis -CH=CH- may be prepared according to the following reaction sequence :

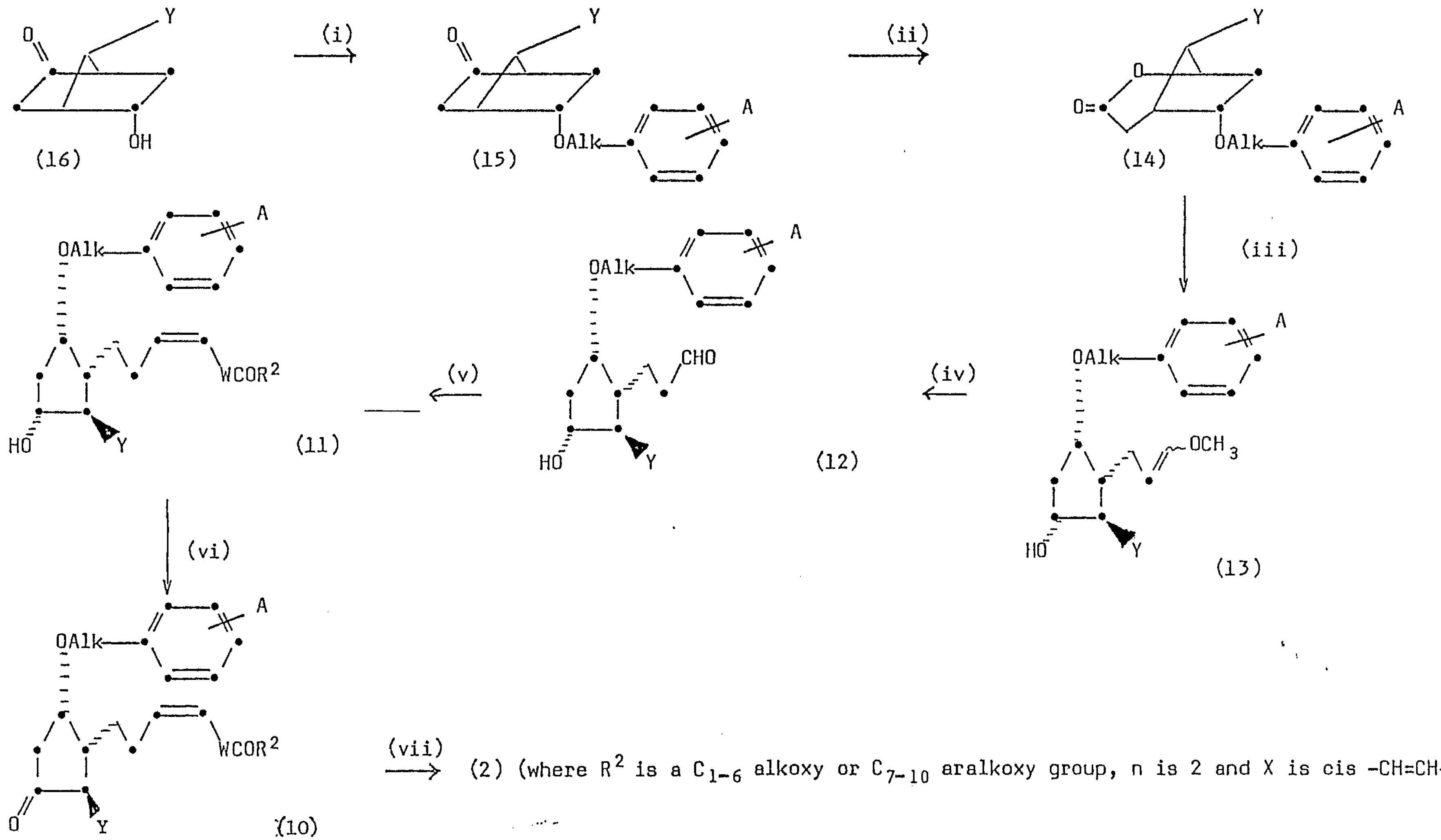


(2) (where $R^2$ is a $C_{1-6}$ alkoxy or $C_{7-10}$ aralkoxy group, n is 2 and X is cis -CH=CH-)

Steps (i)-(vi) may be carried out using reaction conditions similar to those described in GB-A-2075503. Thus, step (i) may be effected by reacting a compound (16) with a reagent

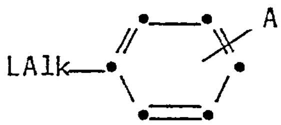 (where L is a leaving group e.g. halogen) under

(where L is a leaving group e.g. halogen) under standard alkylation conditions and, if desired, in the presence of a phase transfer catalyst. Step (ii) is a Baeyer-Villiger oxidation to form the lactone (14). Step (iii) involves reduction (e.g. using diisobutyl aluminium hydride) to give the corresponding lactol which is treated with an appropriate Wittig reagent e.g. a phosphorane of formula $CH_3OCH=PPh_3$ (where Ph is phenyl) to give the enol ether (13). Step (iv) involves hydrolysis (e.g. acid hydrolysis using a mineral acid such as hydrochloric acid) to give the aldehyde (12). Step (v) may be effected by treating the aldehyde (12) with an appropriate Wittig reagent e.g. a phosphorane of formula $Ph_3P=CHWCO_2H$ or a salt thereof, e.g. the potassium salt followed by esterification (eg using a suitable alcohol) to give a compound (11) where $R^1$ is a $C_{1-6}$ alkyl (e.g. methyl) or $C_{7-10}$ aralkyl group. Step (vi) involves oxidation using a suitable oxidising system e.g. dimethyl sulphoxide activated by a suitable electrophilic (such as oxalyl chloride). Step (vii) may be carried out using a variety of reducing systems and conveniently using the general conditions described in EP-A-0234737. Thus, reduction may be effected using a borohydride reducing agent (e.g. sodium borohydride) in the presence of a lanthanide (e.g. cerium trichloride).

The above reaction sequence is particularly suitable for preparing intermediates of formula (2) in which Y is a piperidine ring.

Intermediates of formula (2) in which n is 1 may be prepared according to the general methods described in GB-A-2028805 and GB-A-2070591. Thus, for example, a compound of formula (2) in which n is 1, X is cis $-CH=CH-$ and $R^2$ is a $C_{1-6}$ alkoxy or $C_{7-10}$ aralkoxy group may be prepared from a compound (14) by (i) reduction (eg using diisobutyl aluminium hydride) to give the corresponding lactol (ii) treating the lactol with an appropriate Wittig reagent eg a phosphorane of formula $Ph_3P=CHWCO_2H$ or a salt thereof, eg the potassium salt followed by esterification (eg using a suitable alcohol) to give a compound (17)

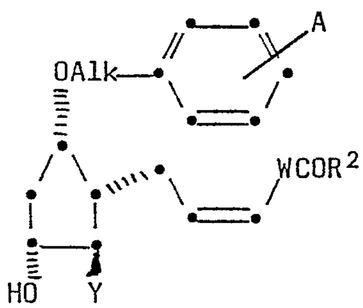

(17)

(where $R^2$ is $C_{1-6}$ alkoxy or $C_{7-10}$ aralkoxy) which is converted into the desired compound of formula (2) by oxidation and then reduction using the conditions described in steps (vi) and (vii) above.

Intermediates of formula (2) in which X is trans $-CH=CH-$ or $-CH_2CH_2-$ may be prepared from intermediates of formula (2) in which X is cis $-CH=CH-$ according to the general processes (e) and (f) described above for preparing compounds of formula (1).

Intermediates of formula (2) in which $R^2$ is hydroxy may be conveniently prepared from the corresponding esters of formula (2) by hydrolysis using the conditions described in process (c) above.

The intermediates of formula (16) are either known compounds or may be prepared by methods analogous to those used for preparing the known compounds of formula (16).

Intermediate compounds of formulae (11), (13), (14), (15) and (17) may be converted to the corresponding intermediates containing the

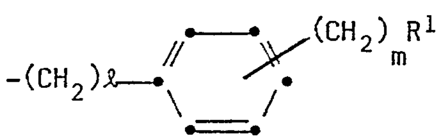

by the methods of process (a) described above or via intermediate boronic acids by the methods of process (b) described above.

When a specific enantiomer of formula (1) is required, starting materials having the desired stereochemical configuration should be used in the above processes.

The following Intermediates and Examples illustrate the invention. All temperatures are in °C. In the following, dried, refers to drying with magnesium sulphate, t.l.c. means thin layer chromatography and $NH_3$ means 0.880 ammonia.

Intermediate 1

[1R-(endo,anti)]-(+)-5-[(4-Bromophenyl)methoxy]-7-(1-piperidinyl)-bicyclo[2.2.1]heptan-2-one,ethanedioate

A mixture of [1R-(endo, anti)]-(+)-5-Hydroxy-7-(1-piperidinyl) bicyclo[2.2.1]heptan-2-one (50g), 4-bromobenzyl bromide (100g), aqueous sodium hydroxide (70%; 125 mℓ), benzyltriethylammonium chloride (5.5g) and dichloromethane (750mℓ) was stirred vigorously for 48h. The mixture was diluted with water (600 mℓ) and the aqueous layer extracted with dichloromethane (200mℓ). The combined organic layer was washed with 0.4N hydrochloric acid (150mℓ) and the aqueous acid layer extracted with dichloromethane (100mℓ). The combined dichloromethane solutions were washed with 8% sodium bicarbonate and dried. A solution of anhydrous oxalic acid (22.5g) and water (8mℓ) in acetone (350mℓ) was then added over 3 min and the precipitated product was filtered off and washed with a mixture of dichloromethane and acetone (4:1; 500mℓ) to give the title compound (91g) as cream crystals m.p. 146-8°.

Intermediate 2

[1R-(endo, anti)]-(-)-6-[(4-Bromophenyl)methoxy]-8-(1-piperidinyl)-2-oxabicyclo[3.2.1]octan-3-one

Intermediate 1 (62.5g) was partitioned between aqueous potassium carbonate (8%; 500mℓ) and dichloromethane (150mℓ; 2x70mℓ). Evaporation of the dried extract gave the free base as an orange oil. The oil was dissolved in a mixture of dichloromethane (300 mℓ), water (100mℓ) and sulphuric acid (2N; 69mℓ) and peracetic acid (ca 38%; 120mℓ) added over 4.5h with stirring and water bath cooling. The mixture was then stirred at room temperature for 46h. The mixture was then added over 80 min to a stirred solution of sodium sulphite (250g) in water (1.3ℓ). After a further 45 min, sodium metabisulphite (100g) was added and the mixture stirred vigorously for 5h. The mixture was cooled in ice and approximately neutralised with 70% sodium hydroxide then basified (pH 8.5-9) with solid potassium carbonate. The aqueous layer was extracted with dichloromethane (3x250mℓ) and the combined organic layers dried and evaporated in vacuo to leave a brown gum. Chromatography on silica gel (Merck 7734; 460g) eluting with dichloromethane (1.8ℓ) then dichloromethane-ether (4:1) gave a cream solid. Crystallisation from isopropanol (50mℓ) gave the title compound (27.9g) as cream crystals m.p. 85-8°. $[\alpha]_D^{20°}$ -33.5° (0.746% in methanol), -20.6° (0.693% in chloroform).

Analysis $C_{19}H_{24}BrNO_3$
Found: C,57.9; H,6.2; N,3.7.
requires C,57.9; H,6.1; N,3.55%.

Intermediate 3

[1R-(1α,2β,3α,4α)]-(+)-4-[(4-Bromophenyl)methoxy]-3-(3-methoxy-2-propenyl)-2-(1-piperidinyl)cyclopentanol

(i) [1R-(endo,anti)]-6-[(4-Bromophenyl)methoxy]-8-(1-piperidinyl)-2-oxabicyclo[3.2.1]octan-3-ol

Diisobutyl aluminium hydride (1M in hexane, 60mℓ) was added over 30 min (internal temp. kept below -73°) to a stirred cooled (dry ice-acetone) solution of Intermediate 2 (6.8g) in dichloromethane (150mℓ) under nitrogen. After a further 1.5h, methanol (100mℓ) was added, keeping the temperature below -70°. The cooling bath was then removed and the mixture stirred for 2.5h. The precipitate was filtered off and washed well with dichloromethane-methanol (1:1; 400mℓ). The filtrate was evaporated in vacuo, the residue re-dissolved in dichloromethane and the solution stirred with anhydrous magnesium sulphate. The solid was filtered off and the filtrate evaporated in vacuo to give the title compound as a yellow gum (7.35g). T.l.c. (silica/ether) Rf ca. 0.1 (streak).

(ii)
[1R-(1α,2β,3α,4α)]-(+)-4-[(4-Bromophenyl)methoxy]-3-(3-methoxy-2-propenyl)-2-(1-piperidinyl)cyclopentanol

A Wittig reagent was prepared from methoxymethyltriphenylphosphonium chloride (25.8g) and potassium tert-butoxide (8.4g) in dry tetrahydrofuran (170mℓ) under nitrogen at 0°. A solution of the product from stage (i) (7.2g) in tetrahydrofuran (60mℓ) was added over 15min, and the solution stirred at 0° for 2.5h. Water (20mℓ) was added and the solvent removed in vacuo to leave a yellow oil. The oil was diluted with water (400mℓ) and extracted with ethyl acetate (150mℓ, 2x100mℓ). Evaporation of the dried extract gave an orange oil. Chromatography on silica gel (Merck 7734; 500g) eluting with ethyl acetate (1ℓ) then ethyl acetate-methanol [(95:5, 1ℓ), (90:10, 1ℓ) then (80:20,1ℓ)] gave an orange oil. The oil was dissolved in ether (50mℓ) and insoluble material filtered off. Evaporation of the ether in vacuo left the title compound (5.83g) as a yellow oil. [α] + 40.8° (0.957% in methanol)
Analysis $C_{21}H_{30}BrNO_3$
Found: C,59.4; H,7.0; N,3.3.
requires C,59.4; H,7.1; N,3.3%.

Intermediate 4

[1R-(1α,2β,3α,5α)]-(+)-5-[(4-Bromophenyl)methoxy]-3-hydroxy-2-(1-piperidinyl)cyclopentanepropanal

A solution of Intermediate 3 (5.63g) in a mixture of acetone (40mℓ) and hydrochloric acid (2N; 25mℓ) was stirred under nitrogen for 2h. The mixture was diluted with sodium carbonate (0.5N, 300mℓ) and the product extracted into dichloromethane (3x50mℓ). Evaporation of the dried extract gave the title compound (5.48g) as a light yellow gum. $[\alpha]_D^{20°}$ + 34.7° (0.831% in methanol)
Analysis $C_{20}H_{28}BrNO_3$
Found: C,58.2; H,7.2; N,3.2.
requires C,58.5; H,6.9; ;N,3.4%.

Intermediate 5

[1R-[1α(Z),2β,3α,5α]]-(+)-Methyl
7-[5-[(4-Bromophenyl)methoxy]-3-hydroxy-2-(1-piperidinyl)cyclopentyl]-4-heptenoate

A Wittig reagent was prepared from 3-carboxypropyl triphenylphosphonium bromide (27.1g) and potassium tert-butoxide (14.6g) in dry tetrahydrofuran (350mℓ) under nitrogen. A solution of Intermediate 4 (5.3g) in tetrahydrofuran (50mℓ) was added over 15min and the mixture stirred a further 2.5h. Water (10mℓ) and methanol (10mℓ) were added and the solvent removed in vacuo. The residue was diluted with water (200mℓ) and washed with ether (2x150mℓ). The aqueous solution was adjusted to pH 7.5 - 8.0 and extracted with dichloromethane (6x50mℓ) then the pH lowered to 7.0 and further extracted with dichloromethane (4x50mℓ). Evaporation of the dried combined extracts gave a yellow foam. The foam was dissolved in methanol (150mℓ) and concentrated sulphuric acid (15mℓ) added with ice cooling. The solution was then stirred at room temperature for 1.75h. The mixture was diluted with ice cold aqueous potassium carbonate (140g in 1.5ℓ) and extracted with dichloromethane (4x100mℓ). Evaporation of the dried extract gave a yellow oil. Chromatography on silica gel (Merck 7734; 440g), eluting with ethyl acetate (1.5ℓ), ethyl acetate-methanol [(98:2, 1ℓ), (95:5, 1ℓ), (90:10, 1.5ℓ) then (80:20: 0.5ℓ)] then ethyl acetate-methanoltriethylamine (79:20:1) gave the title compound (4.29g) as an orange oil. $[\alpha]_D^{20°}$ + 4.9° (0.864% in methanol).
Analysis $C_{25}H_{36}BrNO_4$
Found: C,60.9; H,7.2; N,3.0
requires C,60.7; H,7.3; N,2.8%.

Intermediate 6

[1R-[1α(Z),2β,5α]]-(-)-Methyl
7-[5-[(4-bromophenyl)methoxy]-3-oxo-2-(1-piperidinyl)cyclopentyl]-4-heptenoate

A solution of dimethylsulphoxide (0.25mℓ) in dichloromethane (8mℓ) was added, over 5 min, to a stirred, cooled (-78°) solution of oxalyl chloride (0.26mℓ) in dichloromethane (10mℓ) under nitrogen. After 25 min, a solution of Intermediate 5 (0.98g) in dichloromethane (12mℓ) was added over 5 min. After a further 40 min, triethylamine (1.4mℓ) was added, the cooling bath removed and the mixture allowed to warm to room temperature over 45 min. The mixture was diluted with dichloromethane (100mℓ) and the solution was washed with aqueous citric acid (10%, 50mℓ) and water (50mℓ), dried and evaporated in vacuo to leave a yellow oil.

Chromatography on silica gel (Merck 7734; 70g), set up in ethertriethylamine (99:1), eluting with ether gave the title compound (0.92g) as a pale yellow oil. $[\alpha]_D^{20°}$ -7.0° (0.514% in methanol).
Analysis $C_{25}H_{34}BrNO_4$
Found: C,61.2; H,7.2; N,3.1.
requires C,61.0; H,7.0; N,2.8%.

Intermediate 7

[1R-[1α(Z),2β,3β,5α]]-(+)-Methyl 7-[5-[(4-bromophenyl)methoxy]-3-hydroxy-2-(1-piperidinyl)cyclopentyl]-4-heptenoate

A solution of cerium (III) chloride heptahydrate (0.5g) in methanol (4mℓ) was added to a stirred, cooled (-12°) solution of Intermediate 6 (0.598g) in methanol. After 1 min., sodium borohydride (0.037g) was added in three portions over 1 min. After a further 10 min, the mixture was diluted with sodium carbonate (0.2N; 200mℓ) and extracted with dichloromethane (4x30mℓ). Evaporation of the dried extract gave a colourless oil. Chromatography on silica gel (Merck 7734; 65g) set up in ether:triethylamine (99:1), eluting with ether:triethylamine (99.9:0.1) gave the title compound (0.500g) as a pale yellow oil. $[\alpha]_D^{20°}$ + 76° (0.91% in methanol).
Analysis $C_{25}H_{36}BrNO_4$
Found: C,61.1; H,7.7; N,3.0.
requires C,60.7; H,7.3; N,2.8%.

Intermediate 8

[1R-[1α(Z),2β,3β,5α]]-(+)-7-[5-[(4-Bromophenyl)methoxy]-3-hydroxy-2-(1-piperidinyl)cyclopentyl]-4-heptenoic acid, hydrochloride

A solution of Intermediate 7 (0.61g) in ethanol (12mℓ) and aqueous sodium hydroxide (5N; 2.5mℓ) was stirred under nitrogen for 3h. The ethanol was removed in vacuo, the residue made up to 20mℓ with water then acidified (pH <1) with concentrated hydrochloric acid and extracted with dichloromethane (3x20mℓ). Evaporation of the dried extract gave a cream foam, which was crystallised from dichloromethane-ethyl acetate to give the title compound (0.537g) as white crystals m.p. 145-7°. $[\alpha]_D^{20°}$ + 60° (0.581% in methanol).
Analysis $C_{24}H_{34}BrNO_4$
Found: C,55.7; H,6.8; N,2.6.
requires C,55.8; H,6.8; N,2.7%.

Intermediate 9

[4-[[(1,1-Dimethylethyl)dimethylsilyl]oxy]phenyl]boronic acid, bimolecular anhydride

n-Butyl lithium (1.6M; 9.7mℓ) was added, over 8 min, under nitrogen to a stirred, cooled (internal temp. <-70°) solution of 1-bromo-4-[[(1,1-dimethylethyl)dimethylsilyl]oxy]benzene (4g) in dry tetrahydrofuran (40mℓ). After a further 25 min, the resulting solution was added over 10 min (internal temp. <-66°) to a stirred cooled solution of tri-isopropyl borate (10mℓ) in tetrahydrofuran (40mℓ). The cooling bath was then removed and the mixture stirred for 2h. Water (10mℓ) and, after a further 5 min, pH 6.5 phosphate buffer (100mℓ) and ether (50mℓ) were added and the mixture stirred vigorously for 10 min. The aqueous layer was extracted with ether (2x70mℓ) and the combined organic solutions dried and evaporated in vacuo to leave a white solid. Crystallisation from ether gave the title compound (2.02g) as a white solid m.p. 193-7°. T.l.c. silica (dichloromethane:methanol, 96:4) Rf 0.56
Analysis $C_{24}H_{40}B_2O_5Si_2$
Found: C,59.45; H,8.3.
requires C,59.3; H,8.3%.

Example 1

[1R-[1α(Z),2β,3β,5α]]-(+)-7-[3-Hydroxy-5-[(4′-hydroxy[1,1′-biphenyl]-4-yl)methoxy]-2-(1-piperidinyl)cyclopentyl]-4-heptenoic acid

A mixture of Intermediate 8 (0.4g), Intermediate 9 (0.38g) tetrakis(triphenylphosphine)palladium (O) (0.04g), aqueous sodium carbonate (1M; 5mℓ) and 1,2-dimethoxyethane (12mℓ) was stirred and refluxed under

nitrogen for 6.5h. The mixture was diluted with sodium carbonate (0.5N; 70mℓ) and washed with ether (70mℓ). The ether layer was extracted with sodium carbonate (2N; 10mℓ) and the combined aqueous layer washed with ether (70mℓ). The aqueous layer was adjusted to ca pH6 with concentrated hydrochloric acid, pH 6.5 buffer (20mℓ) added and the solid filtered off. The solid was dissolved in hot methanol (90mℓ), insoluble material filtered off and the filtrate evaporated in vacuo. The residue was crystallised twice from methanol to give the title compound (0.155g) as a fawn solid m.p. 154-6°. T.l.c. silica (dichloromethane:ethanol:$NH_3$, 82:16:2) run twice, Rf 0.08. $[\alpha]_D^{20°}$ + 85° (0.492% in dimethylsulphoxide).

Example 2

[1R(1α(Z),2β,3β,5α)]-(+)-7-[3-Hydroxy-5-[[3 (hydroxymethyl)[1,1'-biphenyl]-4-yl]methoxy]-2-(1-piperidinyl)cyclopentyl]-4-heptenoic acid

A mixture of Intermediate 8 (200mg), 3-(hydroxymethyl)phenyl boronic acid (90mg), tetrakis(triphenylphosphine)palladium (O) (30mg), aqueous sodium carbonate (2N; 3mℓ) and 1,2-dimethoxyethane (6mℓ) was refluxed under nitrogen for 6h and treated with aqueous sodium carbonate (0.5N; 70mℓ). The mixture was washed with diethyl ether (100mℓ), neutralised with hydrochloric acid (2N), diluted with phosphate buffer (pH 6.5; 50mℓ) and brine (50mℓ), and extracted with dichloromethane (4x75mℓ). The dried extract was evaporated and the residue was purified on a column of silica (Merck 9385; 50mℓ) eluted with dichloromethane-methanol-ammonia (85:15:1) to give a gum. The gum was triturated with diethyl ether (10mℓ) and evaporated to give the title compound as a white foam (72mg). T.l.c. silica (dichloromethane:methanol:$NH_3$, 85:15:1) Rf 0.2
Analysis $C_{31}H_{41}NO_5.2H_2O$
Found: C,69.0; H,8.1; N,2.75.
requires C,68.5; H,8.3; N,2.6%.

Example 3

[1R(1α(Z),2β,3β,5α)]-(+)-7-[3-Hydroxy-5-[(3'-hydroxy[1,1'-biphenyl]-4-yl)methoxy]-2-(1-piperidinyl)cyclopentyl]-4-heptenoic acid

A mixture of Intermediate 8 (400mg), 3-hydroxyphenylboronic acid (150mg), tetrakis(triphenylphosphine)palladium (O) (40mg), aqueous sodium carbonate (2N; 5mℓ) and 1,2-dimethoxyethane (12mℓ) was refluxed under nitrogen for 4h and treated with aqueous sodium carbonate (0.5N; 70mℓ). The mixture was washed with diethyl ether (100mℓ), filtered through hyflo, and neutralised with hydrochloric acid (2N). The mixture was extracted with ethyl acetate (2x100mℓ) and dichloromethane (2x100mℓ) and the insoluble residue dissolved in methanol (50mℓ) and added to the combined extracts. The dried organic phase was evaporated and the resulting solid was purified on a column of silica (Merck 9385; 60mℓ) eluted with dichloromethane-methanol-ammonia (85:15:1) to give a gum. The gum was extracted with toluene (2x50mℓ) under reduced pressure and triturated with diethyl ether (50mℓ) to give the title compound as an off-white solid (165mg) m.p. 120-122° (softens 108°). T.l.c. silica (dichloromethane: methanol:$NH_3$, 85:15:1) Rf 0.2 Analysis
$C_{30}H_{39}NO_5.H_2O$
Found: C,70.15; H,8.0; N,3.1.
requires C,70.4; H,8.1; N,2.7%.
The term "active ingredient" as used below refers to a compound of the invention and may be, for example, a compound according to one of the previous examples.

Pharmaceutical Examples

(i) Tablets

These may be prepared by direct compression or wet granulation. The direct compression method is preferred but may not be suitable in all cases as it is dependent upon the dose level and physical characteristics of the active ingredient.

| A. Direct Compression | mg/tablet |
| --- | --- |
| Active Ingredient | 100.00 |
| Microcrystalline Cellulose NF | 298.00 |
| Magnesium Stearate BP | 2.00 |
| | ——— |
| Compression Weight | 400.00mg |

The active ingredient is sieved through a 250μm sieve, blended with the excipients and compressed using 10.0mm punches. Tablets of other strengths may be prepared by altering the compression weight and using punches to suit.

| B. Wet Granulation | mg/tablet |
| --- | --- |
| Active Ingredient | 100.00 |
| Lactose BP | 238.00 |
| Starch BP | 40.00 |
| Pregelatinised Maize Starch BP | 20.00 |
| Magnesium Stearate BP | 2.00 |
| | ——— |
| Compressed Weight | 400.00mg |

The active ingredient is sieved through a 250μm sieve and blended with the lactose, starch and pregelatinised starch. The mixed powders are moistened with purified water, granules are made, dried, screened and blended with the magnesium stearate. The lubricated granules are compressed into tablets as described for the direct compression formula.

The tablets may be film coated with suitable film forming materials, e.g. methyl cellulose or hydroxypropyl methyl cellulose using standard techniques. Alternatively the tablets may be sugar coated.

(ii) Capsules

| | mg/capsule |
| --- | --- |
| Active Ingredient | 100.00 |
| * STA-RX 1500 | 99.00 |
| Magnesium Stearate BP | 1.00 |
| | ——— |
| Fill Weight | 200.00mg |

* A form of directly compressible starch supplied by Colorcorn Ltd., Orpington, Kent.

The active ingredient is sieved through a 250μm sieve and blended with the other materials. The mix is filled into No. 2 hard gelatin capsules using a suitable filling machine. Other doses may be prepared by altering the fill weight and if necessary changing the capsule size to suit.

(iii) Injection for Intravenous Administration

| | |
|---|---|
| Active Ingredient | 50mg |
| Water for injections BP to | 5ml |

Sodium chloride or any other suitable material may be added to adjust the tonicity of the solution and the pH may be adjusted to that of maximum stability of the active ingredient using dilute acid or alkali or by the addition of suitable buffer salts. The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

**Claims**

1. Compounds of the general formula (1)

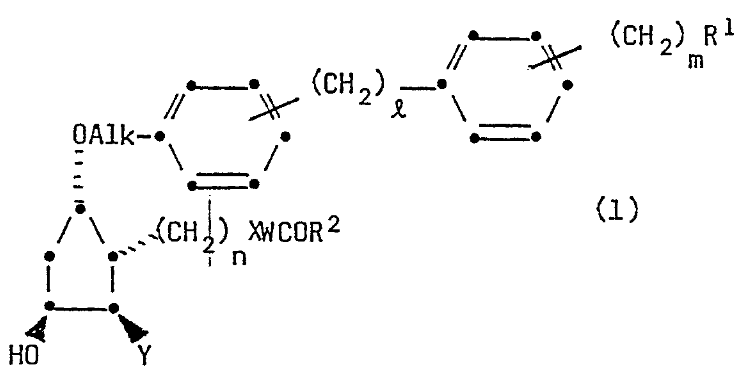

(1)

wherein:

W is straight or branched $C_{1-7}$ alkylene;

X is cis or trans $-CH=CH-$ or $-CH_2CH_2-$;

n is 1 or 2;

Y is a saturated heterocyclic amino group (attached to the cyclopentane ring via the nitrogen atom) which has 5-8 ring members and (a) optionally contains in the ring -O-, -S-, $-SO_2-$, or $-NR^{3a}-$; and/or (b) is optionally substituted by one or more $C_{1-4}$ alkyl groups;

Alk is a straight or branched $C_{1-5}$ alkylene chain;

$\ell$ is zero or 1;

m is zero, 1, 2, 3 or 4;

$R^1$ is a hydroxyl group or a group selected from $-OCOR^3$, $-CO_2R^3$, $-CONR^3R^4$, $-SO_2NR^3R^4$, $-NHCOR^3$, $-NHSO_2R^5$, $-SO_2R^5$, $-SR^5$, $-NR^3R^4$, $-COR^5$, $-NHCONR^3R^4$ and $-NHCSNH_2$ where $R^3$, $R^{3a}$ and $R^4$, which may be the same or different, represent a hydrogen atom or a $C_{1-4}$ alkyl or $C_{7-10}$ aralkyl group and $R^5$ is a $C_{1-4}$ alkyl group;

$R^2$ is

(a) $-NHR^6$ where $R^6$ is a hydrogen atom or a methyl group or a group $-CH_2CO_2H$ or $-CH_2CONH_2$;

(b) $-AR^7$ where A is -O- or -S- and $R^7$ is phenyl [optionally substituted by $C_{1-4}$alkyl, $C_{1-4}$alkoxy, $C_{1-4}$alkanoyl, methylthio, methylsulphinyl, methylsulphonyl, halogen, $-CO_2R^8$ (where $R^8$ is a hydrogen atom, $C_{1-4}$alkyl or phenyl), $-NHCOR^8$, $-CONR^9R^{10}$ (where $R^9$ and $R^{10}$ may be the same or different and are each a hydrogen atom or $C_{1-4}$alkyl), $C_{1-4}$alkylsulphonylamino, formyl, nitro, cyano, phenyl or $-NR^9R^{10}$];

(c) $-OCH_2COR^{11}$ where $R^{11}$ is phenyl (optionally substituted by a halogen atom, $C_{1-4}$alkyl or $C_{1-4}$alkoxy) or a group $-NR^{12}R^{13}$ (where $R^{12}$ is a hydrogen atom or $C_{1-4}$alkyl and $R^{13}$ is a hydrogen

atom, $C_{1-4}$alkyl or phenyl or $NR^{12}R^{13}$ forms a saturated heterocyclic amino group which has 5-7 ring members and optionally contains in the ring -O-);

(d) $-A(CH_2)_pBR^{14}$ where A is as defined above, p is 1-3, B is -O- or -S- and $R^{14}$ is a hydrogen atom, $C_{1-4}$alkyl or a group $-(CH_2)_r$phenyl (where r is 0-3) provided that when p is 1, $R^{14}$ is not a hydrogen atom;

(e) $-A(CH_2)_sR^{15}$ where A is as defined above, s is 2 or 3 and $R^{15}$ is an N-attached $C_{1-4}$dialkylamino, morpholino, piperidino, pyrrolidino, acetylamino or benzoylamino group;

(f) $-OCH(R^{16})_2$ where $R^{16}$ is $-CH_2OH$, $-CHN(CH_3)_2$ or $-CO_2R^{12}$;

(g) $-ACHR^{17}CO_2R^{18}$ where A is as defined above, $R^{17}$ is a hydrogen atom, methyl or phenyl and $R^{18}$ is a hydrogen atom, $C_{1-7}$alkyl, $C_{5-7}$cycloalkyl or phenyl;

(h) $-OCH_2OCOR^{19}$ where $R^{19}$ is $C_{1-4}$alkyl, methoxy or phenyl;

(i) $-OCH_2SCOR^{20}$ where $R^{20}$ is $C_{1-4}$alkyl;

(j) $-AR^{21}$ where A is as defined above and $R^{21}$ is pyridinyl;

(k) $-OR^{22}$ where $R^{22}$ is a hydrogen atom, $C_{1-6}$alkyl, $C_{7-10}$aralkyl, $C_{3-6}$alkenyl, $C_{3-6}$alkynyl, $C_{5-7}$cycloalkyl (optionally substituted by one or more $C_{1-4}$alkyl groups), $-CH_2CCl_3$, furanylmethyl or thienylmethyl;

(l) $-OCH_2CR^{23}R^{24}CH_2OH$ where $R^{23}$ is -OH or $-CH_2OH$ and $R^{24}$ is a hydrogen atom, $C_{1-4}$alkyl or $-CH_2OH$;

(m)

(m) 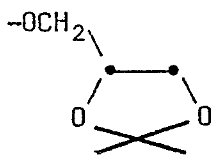 ; or

(n) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; and the physiologically acceptable salts, solvates and cyclodextrin complexes thereof.

2. Compounds as claimed in Claim 1 in which Y is piperidino or hexamethyleneimino.

3. Compounds as claimed in Claim 1 or Claim 2 in which n is 2, X is cis -CH = CH- and W is $-CH_2CH_2-$.

4. Compounds as claimed in any preceding claim in which $R^2$ is hydroxy, methoxy, $-OCH_2OCOCH_3$, $-OCH_2SCH_3$, $-O(CH_2)_3NHCOCH_3$, $-OCH_2CONH_2$, 4-acetamidophenoxy, allyloxy, $-OCH_2CON(CH_3)_2$ or $-OCH_2CH(OH)CH_2OH$.

5. Compounds as claimed in Claim 1 having the formula (1a)

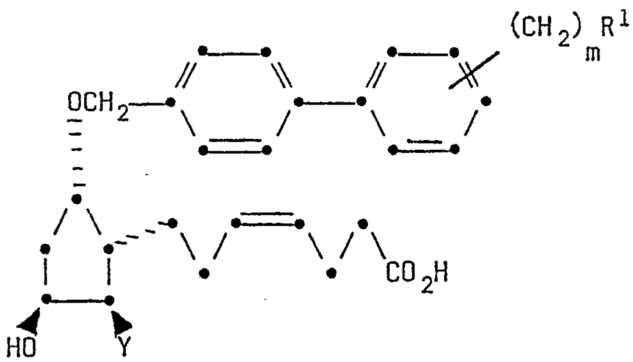

(1a)

wherein m is zero, 1 or 2, Y is piperidino and $R^1$ is -OH, $-CO_2H$, $-CONH_2$, $-NHCOCH_3$, $-NHSO_2CH_3$, $-SO_2NHCH_3$, $-NHCONH_2$ or $-SO_2CH_3$, and the physiologically acceptable salts, solvates and cyclodextrin complexes thereof.

6. Compounds as claimed in Claim 5 in which $R^1$ is -OH and the group $-(CH_2)_mR^1$ is attached at the ortho position of the phenyl group in the rest of the molecule.

7. Compounds as claimed in any preceding claim in which the carbon atom carrying the $-(CH_2)_nXWCOR^2$ group is in the R configuration.

8. A pharmaceutical composition comprising a compound as claimed in any preceding claim together with a pharmaceutical carriers.

9. A process for the preparation of a compound as claimed in Claim 1 which comprises :

(a) reacting a compound of formula (2)

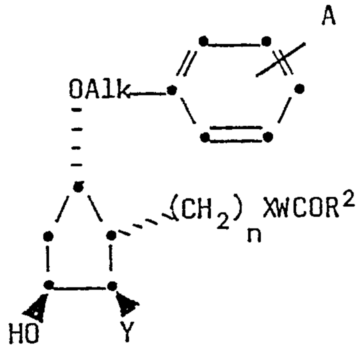

(2)

(where A is a displaceable atom or group) or a salt thereof to replace the moiety A with the group

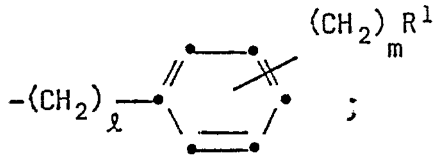

(b) in the preparation of a compound in which $R^2$ is a hydroxyl group and $\ell$ is zero, reacting a compound of formula (8)

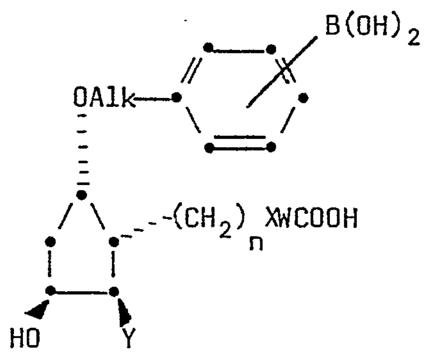

(8)

with a halobenzene of formula (9)

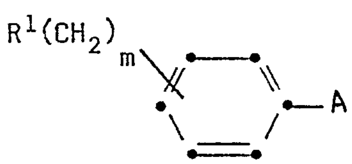

(9)

(where A is a halogen atom);

(c) in the preparation of a compound containing a group -COOH, hydrolysing a corresponding ester;

(d) in the preparation of a compound in which the group $COR^2$ is a carboxylic acid ester or thioester function, esterifying the corresponding carboxylic acid or thioacid;

(e) in the preparation of a compound in which X is trans -CH = CH-, isomerising the corresponding cis compound;

(f) in the preparation of a compound in which X is $-CH_2CH_2-$, catalytically hydrogenating a

corresponding compound in which X is -CH=CH;

(g) in the preparation of a compound in which the group $COR^2$ is a carboxylic acid amide function, amidating the corresponding acid;

(h) in the preparation of a salt, treating a compound of formula (1) with an acid or with a base, or converting one salt into another by ion exchange.

10. Compounds as claimed in Claim 1 for use in human and veterinary medicine.

**Claims for the following contracting States: ES, GR**

1. A process for the preparation of compounds of the general formula (1)

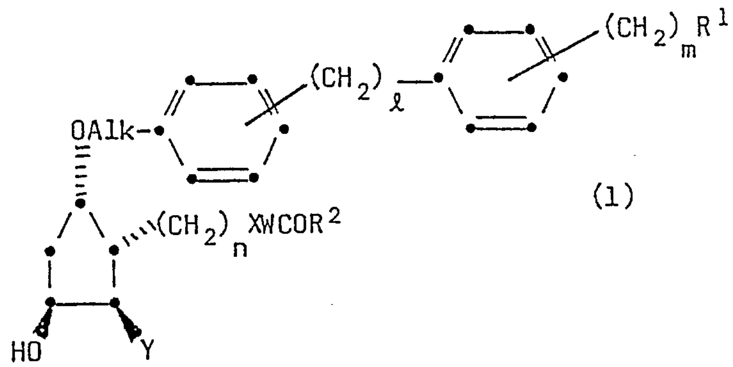

(1)

wherein:

W is straight or branched $C_{1-7}$ alkylene;

X is cis or trans $-CH=CH-$ or $-CH_2CH_2-$;

n is 1 or 2;

Y is a saturated heterocyclic amino group (attached to the cyclopentane ring via the nitrogen atom) which has 5-8 ring members and (a) optionally contains in the ring -O-, -S-, $-SO_2-$, or $-NR^{3a}-$;
and/or (b) is optionally substituted by one or more $C_{1-4}$ alkyl groups;

Alk is a straight or branched $C_{1-5}$ alkylene chain;

$\ell$ is zero or 1;

m is zero, 1, 2, 3 or 4;

$R^1$ is a hydroxyl group or a group selected from $-OCOR^3$, $-CO_2R^3$, $-CONR^3R^4$, $-SO_2NR^3R^4$, $-NHCOR^3$, $-NHSO_2R^5$, $-SO_2R^5$, $-SR^5$, $-NR^3R^4$, $-COR^5$, $-NHCONR^3R^4$ and $-NHCSNH_2$ where $R^3$, $R^{3a}$ and $R^4$, which may be the same or different, represent a hydrogen atom or a $C_{1-4}$ alkyl or $C_{7-10}$ aralkyl group and $R^5$ is a $C_{1-4}$ alkyl group;

$R^2$ is

(a) $-NHR^6$ where $R^6$ is a hydrogen atom or a methyl group or a group $-CH_2CO_2H$ or $-CH_2CONH_2$;

(b) $-AR^7$ where A is -O- or -S- and $R^7$ is phenyl [optionally substituted by $C_{1-4}$alkyl, $C_{1-4}$alkoxy, $C_{1-4}$alkanoyl, methylthio, methylsulphinyl, methylsulphonyl, halogen, $-CO_2R^8$ (where $R^8$ is a hydrogen atom, $C_{1-4}$alkyl or phenyl), $-NHCOR^8$, $-CONR^9R^{10}$ (where $R^9$ and $R^{10}$ may be the same or different and are each a hydrogen atom or $C_{1-4}$alkyl), $C_{1-4}$alkylsulphonylamino, formyl, nitro, cyano, phenyl or $-NR^9R^{10}$];

(c) $-OCH_2COR^{11}$ where $R^{11}$ is phenyl (optionally substituted by a halogen atom, $C_{1-4}$alkyl or $C_{1-4}$alkoxy) or a group $-NR^{12}R^{13}$ (where $R^{12}$ is a hydrogen atom or $C_{1-4}$alkyl and $R^{13}$ is a hydrogen atom, $C_{1-4}$alkyl or phenyl or $NR^{12}R^{13}$ forms a saturated heterocyclic amino group which has 5-7 ring members and optionally contains in the ring -O-);

(d) $-A(CH_2)_pBR^{14}$ where A is as defined above, p is 1-3, B is -O- or -S- and $R^{14}$ is a hydrogen atom, $C_{1-4}$alkyl or a group $-(CH_2)_r$phenyl (where r is 0-3) provided that when p is 1, $R^{14}$ is not a hydrogen atom;

(e) $-A(CH_2)_sR^{15}$ where A is as defined above, s is 2 or 3 and $R^{15}$ is an N-attached $C_{1-4}$dialkylamino, morpholino, piperidino, pyrrolidino, acetylamino or benzoylamino group;

(f) $-OCH(R^{16})_2$ where $R^{16}$ is $-CH_2OH$, $-CHN(CH_3)_2$ or $-CO_2R^{12}$;

(g) $-ACHR^{17}CO_2R^{18}$ where A is as defined above, $R^{17}$ is a hydrogen atom, methyl or phenyl and $R^{18}$ is a hydrogen atom, $C_{1-7}$alkyl, $C_{5-7}$cycloalkyl or phenyl;

(h) $-OCH_2OCOR^{19}$ where $R^{19}$ is $C_{1-4}$alkyl, methoxy or phenyl;

(i) $-OCH_2SCOR^{20}$ where $R^{20}$ is $C_{1-4}$alkyl;

(j) $-AR^{21}$ where A is as defined above and $R^{21}$ is pyridinyl;

(k) $-OR^{22}$ where $R^{22}$ is a hydrogen atom, $C_{1-6}$alkyl, $C_{7-10}$aralkyl, $C_{3-6}$alkenyl, $C_{3-6}$alkynyl, $C_{5-7}$cycloalkyl (optionally substituted by one or more $C_{1-4}$alkyl groups), $-CH_2CCl_3$, furanylmethyl or

thienylmethyl;

(l) $-OCH_2CR^{23}R^{24}CH_2OH$ where $R^{23}$ is $-OH$ or $-CH_2OH$ and $R^{24}$ is a hydrogen atom, $C_{1-4}$alkyl or $-CH_2OH$;

(m)

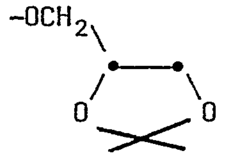

; or

(n) 1-(acetyloxy)ethoxy, (acetyloxy)phenylmethoxy, tetrahydro-5-oxo-2-furanyloxy, tetrahydro-2-oxo-3-furanyloxy, triphenylmethoxy or diphenylmethoxy; and the physiologically acceptable salts, solvates and cyclodextrin complexes thereof, which comprises :

(a) reacting a compound of formula (2)

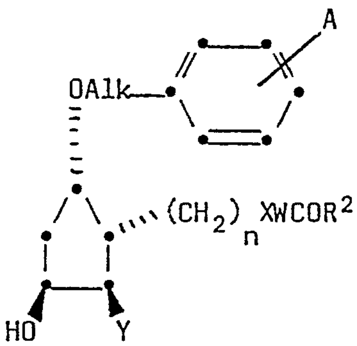

(2)

(where A is a displaceable atom or group) or a salt thereof to replace the moiety A with the group

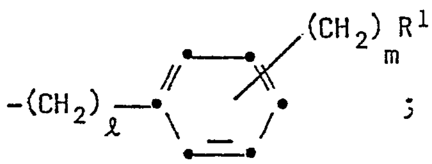

;

(b) in the preparation of a compound in which $R^2$ is a hydroxyl group and $\ell$ is zero, reacting a compound of formula (8)

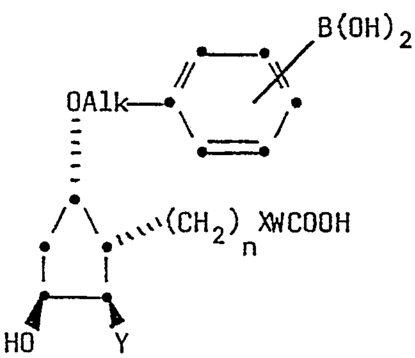

(8)

with a halobenzene of formula (9)

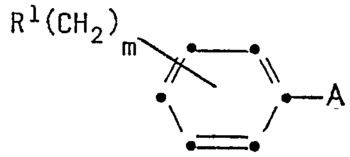

(9)

(where A is a halogen atom);

(c) in the preparation of a compound containing a group -COOH, hydrolysing a corresponding ester;

(d) in the preparation of a compound in which the group $COR^2$ is a carboxylic acid ester or thioester function, esterifying the corresponding carboxylic acid or thioacid;

(e) in the preparation of a compound in which X is trans -CH=CH-, isomerising the corresponding cis compound;

(f) in the preparation of a compound in which X is $-CH_2CH_2-$, catalytically hydrogenating a corresponding compound in which X is -CH=CH;

(g) in the preparation of a compound in which the group $COR^2$ is a carboxylic acid amide function, amidating the corresponding acid;

(h) in the preparation of a salt, treating a compound of formula (1) with an acid or with a base, or converting one salt into another by ion exchange.

2. A process as claimed in Claim 1 in which the product is a compound in which Y is piperidino or hexamethyleneimino.

3. A process as claimed in Claim 1 or Claim 2 in which the product is a compound in which n is 2, X is cis -CH=CH- and W is $-CH_2CH_2-$.

4. A process as claimed in any preceding claim in which the product is a compound in which $R^2$ is hydroxy, methoxy, $-OCH_2OCOCH_3$, $-OCH_2SCH_3$, $-O(CH_2)_3NHCOCH_3$, $-OCH_2CONH_2$, 4-acetamidophenoxy, allyloxy, $-OCH_2CON(CH_3)_2$ or $-OCH_2CH(OH)CH_2OH$.

5. A process as claimed in Claim 1 in which the product has the formula (1a)

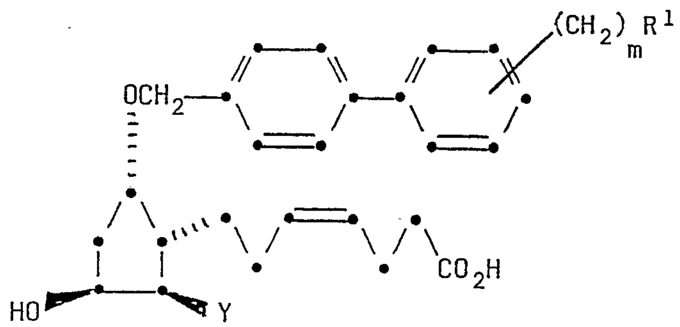

(1a)

and the physiologically acceptable salts, solvates and cyclodextrin complexes thereof, wherein m is zero, 1 or 2, Y is piperidino and $R^1$ is -OH, $-CO_2H$, $-CONH_2$, $-NHCOCH_3$, $-NHSO_2CH_3$, $-SO_2NHCH_3$, $-NHCONH_2$ or $-SO_2CH_3$.

6. A process as claimed in Claim 5 in which the product is a compound of formula (1a) in which $R^1$ is -OH and the group $-(CH_2)_mR^1$ is attached at the ortho position of the phenyl group in the rest of the molecule.

7. A process as claimed in any preceding claim in which the carbon atom carrying the $-(CH_2)_nXWCOR^2$ group is in the R configuration.

8. A process for preparing a pharmaceutical composition comprising mixing a compound as claimed in any preceding claim with one or more pharmaceutical carriers.

9. A process as claimed in Claim 1 for preparing a compound of formula (1) in which $\ell$ is zero which comprises reacting a compound of formula (2) wherein A represents a halogen atom with a compound of formula (3)

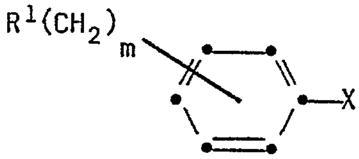

(3)

in which X represents a group $-B(OH)_2$ in the presence of a transition metal catalyst.

10. A process as claimed in Claim 9 carried out in the presence of the catalyst $(Ph_3P)_4Pd$.

European Patent Office

# EUROPEAN SEARCH REPORT

Application number: EP 88305919.8

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 2 070 591 (GLAXO GROUP) * Claims 1,9,10; abstract; examples 1d,3a,4a,7a,7c,9b, 9d,13 * -- | 1-3,5, 8-10 | C 07 D 295/16<br>A 61 K 31/445 |
| A | GB - A - 2 108 116 (GLAXO GROUP) * Claims; abstract * -- | 1-3,5, 8-10 | |
| D,A | CHEMISTRY LETTERS, no. 1, 1986 YAMASHITA et al. "Ullmann-type coupling reaction of aryl trifluoromethansulfonates catalyzed by in situ-generated low valent nickel complexes" pages 407,408 * Totality * -- | 9 | **TECHNICAL FIELDS SEARCHED (Int. Cl.4)** |
| D,A | CHEMISTRY LETTERS, no. 1, 1981 KOSUGI et al. "Palladium catalyzed reaction of hexbutylditin with aryl bromides: preparation of negatively substituted aryltributyltin" pages 829,830 * Totality * -- | 9 | C 07 D 295/00 |

The present search report has been drawn up for all claims

| Place of search | Date of completion of the search | Examiner |
|---|---|---|
| VIENNA | 12-09-1988 | KÖRBER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document